# EUROPEAN PATENT APPLICATION

(11) **EP 1 949 925 A1**
(43) Date of publication of application: **30.07.2008**
(21) Application number: 06841664.3
(22) Date of filing: 24.10.2006
(51) Int. Cl.: A61M 5/32

(54) **DEVICE FOR WITHDRAWING AND CONCEALING THE NEEDLE OF A MEDICAL SYRINGE FOLLOWING INJECTION**

(30) Priority: 07.11.2005 ES 200502705
(71) Applicant: Andersson, Bo Hjalmar, 28016 Madrid (ES)
(72) Inventor: Andersson, Bo Hjalmar, 28016 Madrid (ES)
(74) Representative: PROPI, S.L.
(86) International application number: PCT/ES2006/000589
(87) International publication number: WO 2007/051878

(57) **Abstract**

The invention relates to a device which is applied to the front part of a medical syringe and which is used to withdraw and conceal a used injection needle such that the needle is housed in a safety container. The inventive device is **characterised in that**, once the syringe has been filled, the injection needle disappears following injection by means of either a mechanical or chemical process, said needle being withdrawn and housed in the safety container at the selected pre-programmed moment.

## Description

### OBJECT OF THE INVENTION

The present invention refers to a device that, when applied to the needle of a medical syringe, guarantees that the needle will retract and disappear after the injection has been given.

The device comprising the invention is designed to resolve problems and risks, since it completely eliminates the possibility of re-using syringes with needles that might be infected. It also removes the risk of accidental needle pricks as there is an almost instantaneous retractable mechanism built into the device.

This invention is therefore of great interest to all healthcare professionals.

### BACKGROUND OF THE INVENTION

The device comprising the invention is designed for the satisfactory resolution of the worldwide problems and risks posed by the re-use of hypodermic medical syringes needles infected by different people.

It is general knowledge that the danger transmitting diseases exists when needles are reused. Cases are known of people contracting HIV/AIDS by pricking themselves with infected needles after they have been discarded on beaches and in other public places by drug users. Transmission of serious illnesses occurs in underdeveloped countries through the re-use of the same syringe to inject different people. Health workers are in particular need of safer material.

### DESCRIPTION OF THE PATENT

The device comprising the invention consists of a circular membrane applied to the needle of a syringe so as to obtain support and an adequate, precise axial fixture to the injection needle, while at the same time guaranteeing a suitable position for the device with the needle, when this device is attached to the front part of a medical syringe.

In spite of its structural simplicity, this circular membrane, made of hygienic material, is the base of the device. This circular membrane is made from a material that is solid, rigid and resilient in its dry state. When it comes into contact with any type of fluid, it softens and breaks up to produce an almost immediate retractile effect.

In order to help control the time this softening and disintegration process takes, the circular membrane has a groove, which is also round. Apart from marking the point where breaking will take place, it also regulates the time when it occurs depending on its thickness, which can vary. The break activates the retraction process, along with the disappearance of the needle. At the moment the needle loses its support and axial fixture, a compressed spiral spring around the needle is released and causes the needle to retract and disappear. It is then stored in a safety chamber.

The device comprising the invention, which consists of a circular membrane with a support and axial fixture for the needle, in conjunction with two circular supports, has the special feature of allowing the needle to disappear by means of a mechanical process at the moment the piston plunger reaches the end of its stroke upon completion of the injection.

It should be pointed out that the device also has the special feature, in conjunction with the circular membrane, that in the event of a medical syringe being left with the injection needle and without the piston plunger completing its stroke, for the inappropriate purpose of re-using it, the structure also enables the needle to disappear without any type of foreign intervention, by activating a chemical process. That is to say, the device in the invention guarantees the disappearance of the needle on completion of the injection as the result of a mechanical process and, and if the injection has not been completed, the same effect is brought about by a chemical process.

The functional structure of the device permits and guarantees that, from the moment the syringe is filled with fluid, the chemical process is activated, thereby guaranteeing that by using this device, the needle is always certain to retract.

### DESCRIPTION OF THE DIAGRAMS

To complete the present description and in order to aid better understanding of the characteristics of the invention, and in keeping with an example of practical use, a set of drawings is attached as an integral part of this description, where, by way of illustration and without being at all restrictive, the following are depicted:

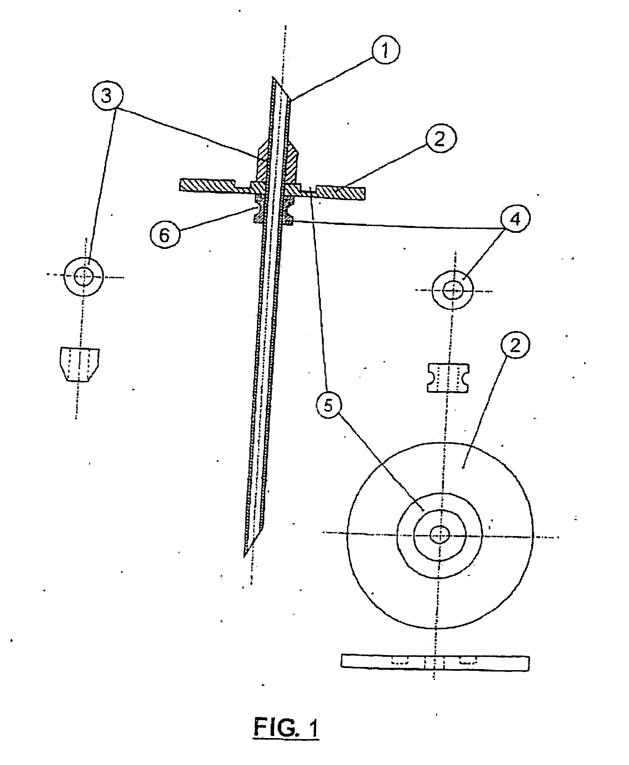

Figure 1 is a schematic diagram of a longitudinal section of the device forming the object of this invention, with the injection needle and the circular membrane with its support and axial fixture, together with the two circular supports that hold it correctly in place. The circular membrane supporting and anchoring the needle has a round groove for weakening the needle, which enables the break point to be set for the needle to retract.

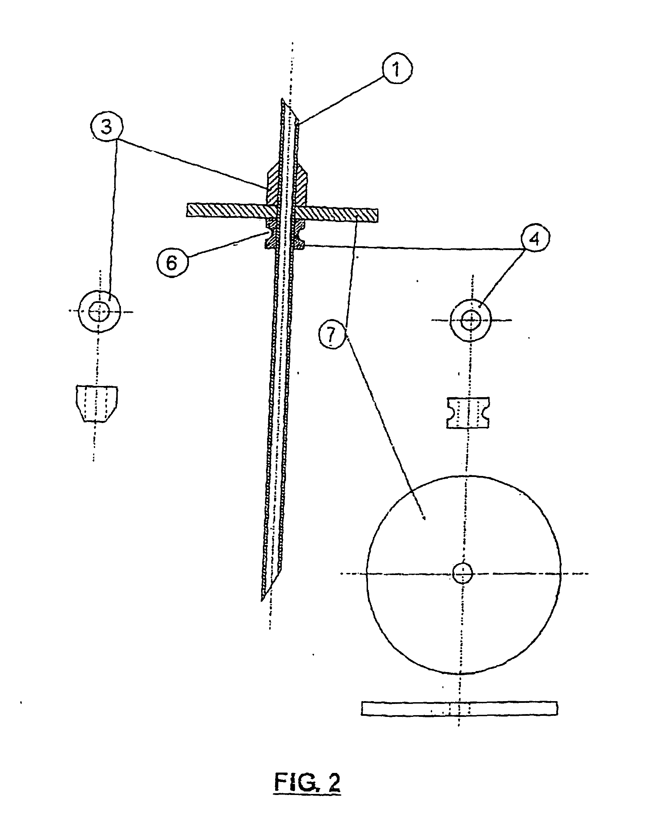

Figure 2 shows a schematic diagram of a longitudinal section of the device comprising the object of this invention with the injection needle and circular membrane with its support and axial fixture, together with two circular supports so that it can be secured properly. The circular membrane, in this case, is completely smooth.

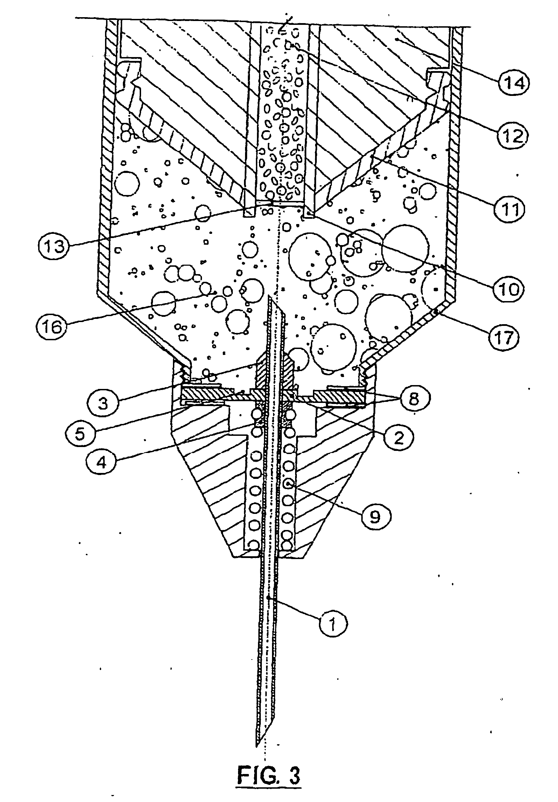

Figure 3 shows a schematic diagram of a longitudinal section of a medical syringe with the device comprising the invention correctly positioned. Position 1 illustrates the device with the syringe and the needle in the position for injection and Position 2 shows how, once the injection has been completed, the needle recedes axially and instantly by means of a mechanical process to position itself in the safety chamber.

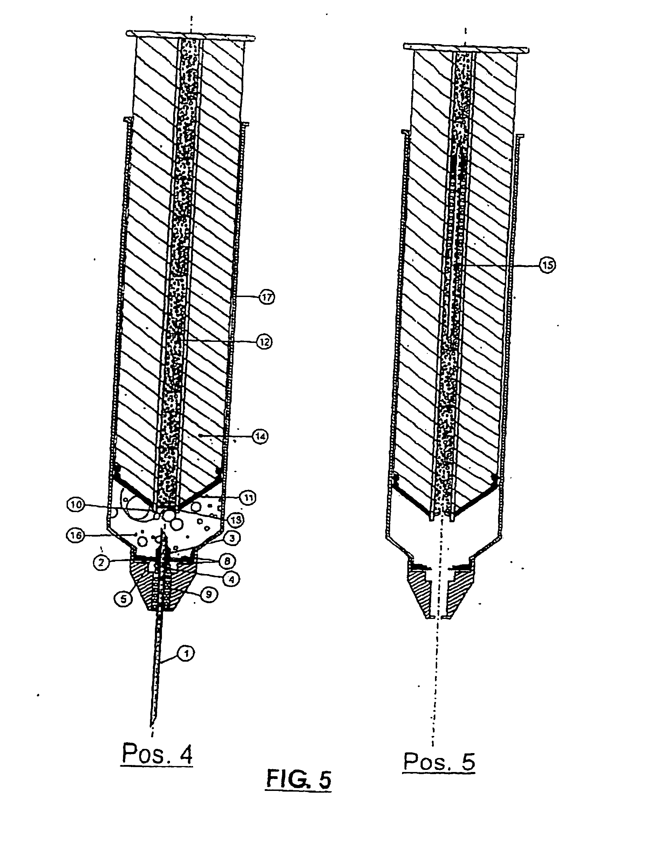

Figure 5 shows a schematic diagram of a longitudinal section of a medical syringe with the device comprising the invention in the correct position. Position 4 illustrates the device with the syringe and the needle in injection position with the piston plunger left without finishing its stroke, for the inappropriate purpose of re-using the syringe. Position 5 shows the special feature of the mechanism of the invention. The inappropriate intention of re-using the syringe proves to be impossible given that at the very moment the syringe is filled with fluid, the suitably-programmed chemical process is activated, with the subsequent automatic retraction of the injection needle and its insertion in the safety chamber.

### PREFERABLE METHOD OF BUILDING THE INVENTION

From the diagrams described above, it can be seen that the device proposed in this invention consists of an injection needle (1) to which is applied, along with two axial supports (3 and 4), a circular membrane made of hygienic material (2), which in its dry state is solid, rigid and resilient, but which softens and disintegrates as a result of the influence of any type of fluid.

This property gives the device comprising the invention its uniqueness; from the moment the syringe is filled with any type of fluid (16), this immediately starts to soften the circular membrane (2), which at the appropriately chosen time, loses its rigidity and axial anchorage causing the needle (1) to recede and disappear automatically due to the pressure exerted by a compressed spiral spring (9) positioned axially around the injection needle (1), until it comes to rest in its final position (15) in the safety chamber (12).

The circular membrane (2), which constitutes a fundamental part of the device comprising the object of this invention, has a round groove (5) in its structure for weakening purposes to fix the break point of same.

The factor regulating the action time of the needle (1) retraction process is based on the difference in thickness of the bottom of the groove (5). In the case of the smooth membrane (7), this regulation is also the consequence of its thickness.

The appropriate attachment of the device comprising the object of the invention to the front of the syringe is guaranteed by the presence and pressure of two circular washers (8). Likewise, the retraction and disappearance of the needle (1) is also instantly guaranteed by the pressure of the circular element (10) protruding from the safety chamber (12) when the piston plunger (11 and 14) reaches the end of its stroke. The displaced needle (15) is then placed in the safety chamber (12). When applied to a medical syringe (17), the device comprising the invention guarantees the retraction and instant disappearance of the needle (1), by means of both the mechanical and also the chemical process, without anybody intervening. The device in the invention guarantees that from the moment the syringe (17) is filled with any type of fluid (16), the needle (1) will disappear, either through the instant mechanical process or else by means of the automatic chemical process, by losing its support and axial fixture, because of the softening and disintegration of the material from which the circular membrane (2) is made, owing to the action of the fluid.

The device comprising the invention, with the circular membrane (2) as its fundamental part, attached to the front of the syringe (17) in the right place, performs its function by means of a piston plunger (11 and 14) which compresses the fluid to be injected. The piston (14) has a circular safety chamber (12) in the centre full of suitable, porous, elastic material to receive the displaced needle (15).

The safety chamber (12) has a membrane (13) at the front made of a flexible, impermeable material, which enables the fluid to be injected (10) to be compressed and enables the needle (1) to recede into the safety chamber (12).

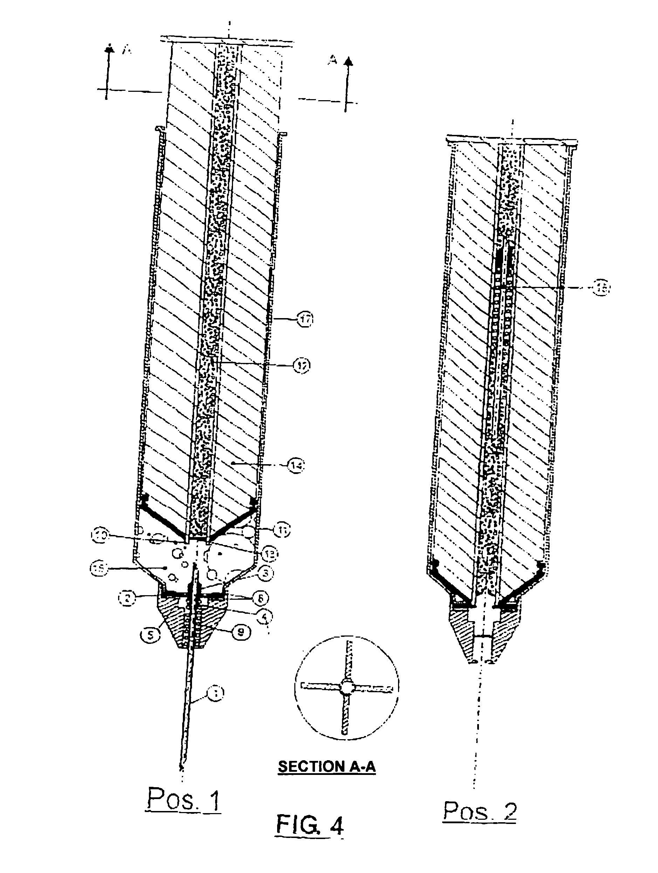

Finally, as illustrated in Figure 4, Positions 1 and 2, the device makes the needle (1) recede and disappear instantly by means of a mechanical process.

Figure 5, Positions 4 and 5, illustrates how the needle (1) recedes and disappears automatically, without involvement by individuals, with the used needle (15) being housed in its safety chamber (12), after having lost its support and axial fixture on account of the softening of the circular membrane (2) caused by the influence of the fluid (16).

## Claims

1. Device for withdrawing and concealing a needle attached to a medical syringe, **characterised in that** it operates mechanically at the end of the injection and also chemically, spontaneously without any manual intervention.

2. The device as claimed in Claim 1 is **characterised in that** it has a circular membrane (2) applied in an axial position to the needle (1) of the medicinal syringe (17).

3. The circular membrane (2), as claimed in Claim 2, is **characterised in that** it is formed from a material that is rigid and strong when in a dry and solid state, but when in contact with any liquid (16) becomes soft and disintegrates.

4. The circular membrane (2) as claimed in Claims 2 and 3 is also **characterised in that** it has a circular groove in its structure, which weakens it and also serves to set its breaking point. The difference in thickness of the bottom of the circular groove (5) and the circular membrane (2) controls and decides the moment of disintegration and breakage of the material thereof, and with it, the withdrawal and disappearance of the needle (1) caused by a spiral spring (9) fitted around the needle (1) and pressure pressed.

5. The structure of the circular membrane (2) as claimed in the three previous claims is **characterised in that** it allows and guarantees that, from the moment the syringe (17) is filled with any liquid (16), the chemical process initiates and automatically causes the withdrawal of the needle (1) such that

6. when the needle loses its support and axial grip and the spiral spring (9) actuates, the needle (1) withdraws into the safety device (12).

7. The device is also **characterised in that** when fitted to the front part of a medical syringe (17), it guarantees the withdrawal and disappearance of the injecting needle (1) when the plunger (11-14) reaches the end of its course, once the injection is complete, and places the needle (15) into the safety device (12).

8. Likewise, the device of this invention, as claimed in Claim 1, is **characterised in that** it causes the injection needle (1) to withdraw by means of a chemical or mechanical process.

9. The mechanical processes consists in the breakage of the circular supporting and gripping membrane (2) by the plunger (10-11 and 14) at the end of its course, once the injection is complete.

10. The device, as claimed in Claim 1, is also **characterised in that** the circular membrane (7), an integral and fundamental part of the device attached to the injection needle (1), is composed of a material that in a dry state is solid, rigid and firm, but that on contact with any liquid (16) becomes soft and disintegrates, causing the injection needle (1) to lose is axial support, causing it to withdraw into the safety device (12), but in contrast to Claim 4, the circular membrane is smooth and without a circular groove.

## Amended claims

### Amended claims under Art. 19.1 PCT

**1.** Device for withdrawing and concealing the needle of a medical syringe following injection, of the type that is based on the use of a material for joining the needle to the body of the syringe that is rigid and strong when in a solid, dry state, but which softens and disintegrates when it comes into contact with a liquid, **characterised in that** said material consists of a membrane (2) that is appropriately secured by its perimeter to the body of the syringe and is solidly joined to the needle (1) with the aid of a pair of axial supports (3) and (4) that are solidly joined to the needle (1) on both sides of the membrane (2), said supports (3) and (4) presenting a smaller diameter than that of the safety container (12) that is designed to house the needle (1) after the syringe is used, so that a spiral spring (9) mounted coaxially on the needle (1) acts on the front support (4) and causes the needle with its supports to retract into the syringe when the membrane (2) is dissolved by having come into contact with the injectable liquid (16).

**2.** Device for withdrawing and concealing the needle of a medical syringe following injection, according to claim 1, **characterised in that** the membrane (2) has a circular groove (5) that is coaxial with the needle (1), which determines a weakening of said membrane that ensures the breakage thereof at the level of said groove (5), the diameter of the membrane sector (2) being such that it remains joined to the needle (1) after the membrane has broken, and it can also enter the safety container (12) that receives the needle.
